# EUROPEAN PATENT APPLICATION

(11) **EP 1 627 912 A1**
(43) Date of publication of application: **22.02.2006**
(21) Application number: 04734133.4
(22) Date of filing: 20.05.2004
(51) Int. Cl.: C12N 5/00, C12N 15/09

(54) **PREPARATION OF ENDODERMAL STEM CELLS**

(30) Priority: 20.05.2003 JP 2003142303
(71) Applicant: RIKEN, Wako-shi, Saitama 351-0198 (JP)
(72) Inventor: NISHIKAWA, Shinichi, Kobe-shi, Hyogo 650-0003 (JP); ERA, Takumi, Kobe-shi, Hyogo 658-0084 (JP)
(74) Representative: Schlich, George William
(86) International application number: PCT/JP2004/007227
(87) International publication number: WO 2004/104184

(57) **Abstract**

It is intended to prepare endodermal stem cells by differentiation from multipotential stem cells followed by isolation. This object can be achieved by separating various endodermal stem cells having been differentiated from multipotential stem cells with the use of, as indications, a specific cell surface marker, an organizer-specific marker and the expression manner of E-cadherin.

## Description

### TECHNICAL FIELD

The present invention relates to endodermal stem cells which are highly purified from mixtures of intermediate stem cells which have been differentiated in vitro from multipotential stem cells and to methods for preparing stem cells of interest.

### BACKGROUND OF THE INVENTION

In mammals, three germ layers, referred to as endoderm, mesoderm, and ectoderm, are formed with dynamic morphological changes during the gastrulation. Endoderm is a tissue which differentiates into stomach, intestinal tract, liver, spleen, large bowel in the future. Similarly to other tissues, endoderm is an important tissue, and thus development of systems providing for the separation and proliferation of endoderm is a major subject for development of drugs and of cells producing hormones and bioactive substances, such as insulin producing cells, and also for clinical applications.

Some endodermal tissues have been proven to differentiate from the organizer region in mice. Organizer is a collective term for a group of cellular tissues playing a leading role in the formation of the body axis, whose existence was reported in 1924 by Spemann and Mangold, et al. That is, the transplantation of the dorsal blastoporal lip of an embryo into another results in the formation of a secondary axis at the implanted site. Subsequent studies have revealed that the organizer exists beyond species, such as zebrafish, chicken, and others. It has been shown that in mice, the node having a specific tissue morphology and formed at the apex portion of the primitive streak is responsible in part for this role (Cell, 1999, Vol. 196, pp. 195-209). Node cells are not only involved in the formation of the body axis, but also differentiate in the future into a tissue referred to as axial mesendoderm, from which mesodermal and endodermal tissues mainly differentiate and proliferate.

Mouse have a small number per individual of organizer-specific cells, for which reason, it is difficult to deal with these cells, and thus many aspects are unclear about the molecular biological mechanism concerning differentiation and proliferation of organizer-related cells. Therefore, in order to solve the problem that only a limited number of these cells can be isolated, the inventors have conducted studies for purifying these cells of which direct isolation from individuals is difficult, employing embryonic stem cells.

Embryonic stem (ES) cells are cells present in an early embryo and having the pluripotentiality of differentiation, and differentiate into different cells, including also germ cells, when injected into other blastcysts. Mouse embryonic stem cells, on which studies are most advanced, are cells possessing the pluripotentiality and self-replication capability which are established from the inner cell mass within the blastodermic vesicle at the day 3.5 of the development. These cells are capable of maintaining their proliferation, while retaining their undifferentiated states, only by adding serum and a growth factor, referred to as leukemia inhibitory factor (LIF), to a usual culture medium. Mouse embryonic stem cells can re-differentiate in vivo into all tissue cells by injecting them into a blastodermic vesicle at the day 3.5 of the development and returning the blastodermic vesicle into the mother body and are used for the production of chimera and knock-out mice. In addition, it has been recently possible to manipulate embryonic stem cells in vitro so as to differentiate them into various mature tissue cells (for example, Shinichi NISHIKAWA et al., Development, 1998, No. 125, pp. 1747-1757; Toru NAKANO et al., Science, 1994, No. 265, pp. 1098-1101; Takumi ERA et al., Blood, 2000, No. 95, pp. 870-878). Because of the pluripotentiality of differentiation and easy handling as described above of embryonic stem cells, they are expected to be utilized in future medical treatment as materials for implantation treatments employing cells.

It has been demonstrated in studies by the inventors and other groups that the appearance of mature cells is brought about when embryonic stem cells were forced into in vitro differentiation. Although it is supposed, at present, that embryonic stem cells arrive at completely mature cells via stem cells that are at various stages of differentiation (intermediate stem cells), the process of their differentiation still remains unclear in many respects.

In addition, there is no reporting, until now, of the separation of highly pure endodermal cells directly from these embryonic stem cells, and thus its development is urgently needed.

### DISCLOSURE OF THE INVENTION

The present inventors have carried out identification, purification, and analysis of the differentiation capability of endodermal cells, employing in vitro differentiation systems of embryonic stem cells. Unfortunately, until today, there are not found cell surface markers specific for the endoderm. For this reason, the inventors made embryonic stem cells in which the green fluorescence protein (GFP) gene, as a marker gene, was knocked-in the goosecoid (Gsc) gene, a gene expressed specifically in the above-mentioned organizer, and attempted to identify and purify endodermal cells. In consequence, it was found that endodermal stem cells which have been differentiated from embryonic stem cells express organizer-specific markers and E-cadherin, leading to the completion of the invention based on this finding.

Therefore, the present invention relates to:
(1) an endodermal stem cell which has been differentiated in vitro from a multipotential stem cell, wherein the cell is organizer-specific-marker positive and E-cadherin positive; preferably,
   the endodermal stem cell wherein the multipotential stem cell is an embryonic stem cell, wherein the organizer-specific marker is goosecoid, wherein a label protein is green fluorescence protein (GFP), and/or wherein the label protein is fused to the organizer-specific marker;
(2) a method for preparing an endodermal stem cell, which comprises the steps of a) culturing multipotential stem cells, and b) selecting and separating cells expressing an organizer-specific marker and E-cadherin; preferably, the method wherein a cell sorter is employed at the selection step; further preferably, the method wherein the multipotential stem cells are cultured in the presence of activin in a serum-free medium on a culture plate coated with collagen IV, thereby differentiating the multipotential stem cells into endodermal stem cells;
(3) a method for differentiating an endodermal stem cell of the present invention to prepare an endodermal cell of interest; preferably, the method wherein the endodermal stem cells are cultured in the presence of activin in a serum-free medium on a culture plate coated with collagen IV, thereby differentiating the endodermal stem cells into endodermal cells; further preferably, the method wherein the endodermal stem cells are cultured in the presence of bFGF (basic fibroblast growth factor) with activin, thereby differentiating the endodermal stem cells into endodermal cells; more preferably, the method wherein activin A is used as activin; and endodermal cells obtained by these preparation methods; and
(4) a method for preparing a stem cell of interest from a multipotential stem cell, which comprises the steps of a) introducing a gene of a label protein in the genome of a multipotential stem cell so as to be adapted to the expression system of a predetermined marker gene, such that said label protein is expressed instead of or together with the predetermined marker gene, and b) selecting and separating stem cells of interest using the label of said label protein as an indicator; preferably, the method wherein the multipotential stem cell is an embryonic stem cell and the label protein is green fluorescent protein.

### BRIEF DESCRIPTION OF THE DRAWINGS

Fig. 1A is a scheme for the production of an embryonic stem cell having a knocked-in goosecoid gene.
Fig. 1B shows the results of southern blotting analysis of homologous recombinant embryonic stem cells employing the GSC gene as a probe.
Fig. 2 represents the results of cell selection showing the appearance of GFP-positive cells by inducing the differentiation and of RT-PCR showing that goosecoid is expressed only in GFP-positive cells.
Fig. 3 shows the pattern of gene expression in the GFP-positive cells obtained by cell selection.
Fig. 4 shows the results of inducing the differentiation of Gsc-GFP-positive cells in a serum-free medium supplemented with activin A.
Fig. 5 shows the results that the percentage of GFP-positive cells is increased depending on the concentration of activin A.
Fig. 6 shows effects of BMP-4 and bFGF on inducing the differentiation of GFP-positive cells.
Fig. 7 shows the expression pattern of E-cadherin in GFP-positive cells.
Fig. 8A shows the differentiation of epithelioid cells from GFP-positive, E-cadherin-positive cells, and their cellular morphology.
Fig. 8B shows the expression of cell-linage specific genes in epithelioid cells from GFP-positive, E-cadherin-positive cells.

### BEST MODE FOR CARRYING OUT THE INVENTION

(1) The present invention provides an endodermal stem cell which has been differentiated in vitro from a multipotential stem cell, wherein the cell is organizer-specific-marker positive and E-cadherin positive.
   As used herein, the term "multipotential stem cell" means a stem cell which is capable of self-replication and possesses the capability of differentiating into at least one cell selected from an ectodermal, a mesodermal and an endodermal stem cell, and includes an embryonic stem (ES) cell, an embryonic germ (EG) cell, an embryonal carcinoma (EC) cell, a multipotent adult progenitor (MAP) cell, an adult pluripotent stem (APS) cell, a bone marrow stem cell, and others. Use can be made of multipotential stem cells derived from a variety of animals such as mammals, including human, monkey, mouse, rat, hamster, rabbit, guinea pig, caw, pig, dog, horse, cat, goat, sheep; birds, reptiles, and others. Usually, multipotential stem cells form mammals are used.
   As used herein, the term "embryonic stem cell" means a cell present in an early embryo and having the pluripotentiality of differentiation and which can differentiate into different cells, including also germ cells, when injected into other blastcysts. In the present invention, use may be made of embryonic stem cells which have been freshly established from the inner cell mass within the blastodermic vesicle, or of already established cell lines.
   As used herein, the term "endodermal stem cell" means an intermediate stem cell which differentiates into a cell belonging to the endodermal tissue but which does not differentiate into a cell belonging to the mesodermal and ectodermal tissues. In the development of a mouse within the mother body, presumptive mesodermal cells which have left from a specified region of the epithelial tissue forming the blastula penetrate into the inside of the embryo and move between the presumptive ectoderm and endoderm to form the presumptive mesoderm, during the gastrulation occurring from day 6.5 to day 7.5 of the development. Cells constituting these presumptive ectoderm, endoderm, and mesoderm are ectodermal, endodermal, and mesodermal stem cells, respectively.
   As used herein, the term "intermediate stem cell" means a cell which is at an advanced stage of differentiation of a multipotential stem cell and which is any one of an ectodermal, mesodermal, or endodermal stem cell, or mixtures thereof. Therefore, an intermediate stem cell may be expressed as a cell possessing the capability of differentiating into any one of an ectodermal cell, a mesodermal cell, or an endodermal cell.
   Cell implantation treatments require differentiating embryonic stem cells in vitro, bringing about the appearance of intermediate stem cells differentiating into a given germ layer, and further purifying them to prepare intermediate stem cells of one kind. As cell materials utilized for cell implantation treatments, intermediate stem cells are of greater value in use than mature cells in that:
   1. Mature cells of most tissues have low capabilities of proliferation, whereas intermediate stem cells have much higher capabilities of in vitro proliferation. This means that making up of appropriate conditions could force them into in vitro proliferation.
   2. Intermediate stem cells of one kind differentiate into various kinds of mature cells, and therefore a smaller number of cells could achieve higher results, when the therapeutic effect is compared.

   As used herein, the term "organizer-specific marker" means a protein which is expressed specifically in the organizer, and includes, in the case of mice, goosecoid (Gsc), HNF-3β, and Lim1. The goosecoid gene is a transcription factor having homeobox domains and it has been shown that among genes expressed in the organizer, this gene is expressed specifically in the organizer (in Principles of Development, Lewis Wolpert, OXFORD Press, 2002, pp. 105). The number of organizer-specific marker to be use d in the present invention may be one, or two or more.
   HNF-3β, an organizer-specific marker, is described in Ang, S.-L. and Rossant, J. (1994), Cell, 78, 561-574; Weinstein, D. C. et al., (1994), Cell, 78, 575-588; and others; Lim1 is described in Shawlot, W. et al. (1995) Nature, 374, 425-430.
   E-cadherin is a transmembrane-typed membrane protein responsible for adhesion between cells (Annu. Rev. Cell, Dev. Biol., 1997:13, 119-146) and it is known that E-Cad-deficient mice are embryonically lethal at very early stages.
   In this aspect, preference is given to cells in which a label protein has been fused to an organizer-specific marker, or alternatively a marker gene has been substituted with a gene of a label protein (gene knock-out method). As a label protein can be utilized green fluorescence protein (GFP), or any protein which can be used on fluorescence activated cell sorters (FACS's), for example, dsRSD. Fusions can be made by means of procedures well-known to those skilled in the art, for example, homologous recombination and gene introducing.
   As used herein, the term "positive (+)" for a given molecule means that a cell expresses the molecule, and "negative (-)" means that no expression occurs. It is possible to determine whether a cell expresses a given molecule or not, for example, by FACS.
   As used herein, the term "in vitro" means that a reaction or culture is carried out in the outside of a living body, including an embryo. When cells are cultured and/or differentiated in vitro, all the media, reagents, and containers suitable for the growth of the cells can be used. "In vivo" in the specification means that a reaction or culture is performed in the inside of the living body, including an embryo, or that a certain phenomenon takes place within a living body.
(2) As another aspect, the present invention provides a method for preparing an endodermal stem cell, which comprises the steps of a) culturing multipotential stem cells, and b) selecting and separating cells expressing an organizer-specific marker and E-cadherin.
   In the preparation method described above, it is preferable that a label protein is fused to an organizer-specific marker or a marker gene is substituted with a gene of a label protein (gene knockout method). In this case, cells of interest are selected by a cell sorter, preferably by a fluorescence activated cell sorter (FACS), using the label of the label protein as an indicator.
   An FACS is usually provided with a flow cytometer, a laser generating unit, an optics, a data processing unit, and a cell sorting unit. The function of a FACS is automatic separation of fluorescently labeled cells and computer analysis of intensities of their fluorescence. By a FACS, cells fluorescently labeled with a specific substance are irradiated with a laser beam on the way of a narrow flowpath, followed by measuring signal information of scattered lights (forward and lateral scattered lights) and fluorescence on every individual cells and displaying its result, for example, as a frequency distribution, so that cells yielding a specific signal information can be sorted. FACS equipment is commercially available from Becton-Dickinson and others, and can be operated by those skilled in the art according to the manufacture's instructions.
   The positivity for E-cadherin is determined by using an anti-E-Cad antibody. In addition, a fluorescence activated cell sorter (FACS) can be used in order to select cells on the basis of its molecular expression.
   Antibodies for use in the present invention may be either polyclonal or monoclonal antibodies, with monoclonal antibodies being preferable when used on a FACS. Such antibodies can be produced by those skilled in the art with reference to the methods described in the Examples, while commercially available antibodies may be used. An anti-E-cadherin monoclonal antibody is commercially available from Takara Shuzo (article No. M108), or can be easily prepared by procedures well known to those skilled in the art.
   The positivity for E-cadherin can be also selected using the presence of intracellular mRNA of E-cadherin as an indicator.
   For culturing embryonic stem cells, use is made of media having compositions suitable for the purpose of maintaining or differentiating the cells. Media for maintaining embryonic stem cells usually are minimal media for cell culture supplemented with serum, LIF, L-glutamine, 2-mercaptoethanol, and others. One example of such compositions is 85% KNOCKOUT D-MEM, 15% FBS, 10⁻⁴ M 2-ME, 2 mM L-glutamine, 0.1 mM NEAA, and 1000 U/ml LIF. Media for differentiating embryonic stem cells usually are minimal media for cell culture supplemented with serum, L-glutamine, 2-mercaptoethanol, and others, and do not contain LIF. One example of such compositions is 90% α-MEM, 10% FBS, 5x10⁻⁵ M 2-ME, 2 mM L-glutamine. To each medium can be added other substances useful for culturing, such as antibiotics, and substitutes having equal functions may be used instead of the respective components. The respective components of a medium are sterilized and used by their appropriate ways.
   Specific procedures in the method for culturing embryonic stem cells according to the present invention can be carried out following procedures and conditions well-known in the art. For example, decision can be made as appropriate, taking into consideration the descriptions, for example, in Norio NAKATSUJI, ed.: Zikken Igaku (Experimental Medicine), Suppl. Vol., Experimental Course 4 in the Post-Genome Era, "Stem Cell and Clone Research Protocols", Yodosha Co., Ltd. (2001); Hogan, G. et al., ed., Mouse Embryo Manipulations: A Laboratory Manual, Cold Spring Harbor Laboratory Press, Plainview, NY (1994); Robertson, E. J. ed., Teratocarcinoma and Embryonic Stem Cells, A Practical Approach, IRL Press, Oxford, UK (1987).
   Certain embodiments of the present invention include a step of differentiating embryonic stem cells into endodermal stem cells. Embryonic stem cells can be differentiated by any of the methods known in the art, and are typically cultured using a serum-free medium without LIF in the presence of activin in containers coated with collagen IV. Activin used here is a peptidic cell-growth/differentiation factor having a size of 24 kD and belonging to the TGF-β (transforming growth factor-beta) family and constitutes a dimmer having two beta-subunits via S-S linkage (Ling, N. et al., (1986) Nature, 321, 779-782; Vale, W. et al., (1986) Nature, 321, 776-779). In the present invention, any of activins A, B, C, and D can be used, or activins from any animal, such as humans and mice can be also used, and they are commercially available from R & D. Among them, activin A is preferably employed. It is preferable to employ activin from the same animal species as the species from which multipotential stem cells used are derived. For example, it is preferable that human activin A is employed when multipotential stem cells from humans is used as the starting material. The concentration of activin is preferably 10 ng/ml or higher, with 10 ng/ml being the most preferable. At lower concentrations, the induction will be also brought about, but the percentage of positive cells is small.
   In addition, it is possible to differentiate multipotential stem cells more preferably into endodermal stem cells, in the case where the multipotential stem cells are cultured in the presence of bFGF (basic fibroblast growth factor) with activin. BFGF is available from R & D. The concentration of b-FGF is preferably 10 ng/ml or higher, with 10 ng/ml being the most preferable.
   When embryonic stem cells are cultured under these conditions, mouse embryonic stem cells will reach an optimal number of endodermal stem cells which is suitable for the purification according to the present invention, usually at day 4 to day 6. Alternatively, it is possible to allow embryonic stem cells to differentiate into endodermal stem cells also by the embryoid body formation method which has been carried out in the past. In addition, although it is at low efficiencies, substituting of gelatin, fibronectin, or the like for collagen IV allows the differentiation of embryonic stem cells into endodermal stem cells (see, for example, Wiles, M. et al., Development, 111, 259-267, 1991).
(3) As another aspect, the present invention provides a method for differentiating an endodermal stem cell of the present invention to prepare an endodermal cell of interest, and an endodermal cell obtained by this preparation method.
   This method for differentiation may be carried out in similar procedures as described above.
   Endodermal cells which are obtained by differentiating endodermal stem cells of the present invention are cells which differentiate into stomach, duodenum, intestinal tract, liver, spleen, and large bowel in the future, which can find use in development of drugs, and as cells producing hormones, bioactive substances, and others, such as insulin producing cells, and for clinical applications.
(4) As another aspect, the present invention provides a method for preparing an intermediate stem cell of interest from a multipotential stem cell. An intermediate stem cell of interest in this embodiment has the capability of differentiating into any one of an ectodermal cell, a mesodermal cell, or an endodermal cell. In this method, a) a gene of a label protein is introduced in the genome of a multipotential stem cell so as to be adapted to the expression system of a predetermined marker gene, such that said label protein is expressed instead of or together with the predetermined marker gene. As a predetermined marker gene, use can be made of markers which are specifically expressed in any one of an ectodermal, a mesodermal, and an endodermal stem cell, for example, the specific marker gene SOX1 in the case of the cells being ectodermal stem cells, the specific marker gene Brachyury or Mesogenin1 in the case of mesodermal stem cells, and the specific marker gene GATA4 or SOX17 in the case of the cells being endodermal stem cells. For the introduction of a gene of a label protein, homologous recombination is usually employed, and additionally transgenic methods in which promoters are linked can be used.

Subsequently, an intermediate stem cell of interest is selected and separated using the label of said label protein as an indicator. In this aspect, it is preferable that the multipotential stem cell is an embryonic stem cell and the label protein is green fluorescence protein.

The method in this aspect is applicable to methods of preparing precursor cells of intermediate stem cells (cells which are at stages capable of differentiating into any two of ectodermal, mesodermal, or endodermal stem cells) from multipotential stem cells. Therefore, the present invention also includes a method characterized by a) introducing a gene of a label protein in the genome of a multipotential stem cell so as to be adapted to the expression system of a predetermined marker gene, such that said label protein is expressed instead of or together with the predetermined marker gene, and b) selecting and separating intermediate stem cells of interest using the label of said label protein as an indicator.

The following examples represent an embodiment of the present invention, and at the same time demonstrates that endodermal stem cells prepared by the method of the present invention have the capability of differentiating into mature cells.

### EXAMPLES

### Reference Example 1: Maintaining of Embryonic Stem Cells

### a. Materials

For maintaining embryonic stem cells, reagents and equipment indicated in Table 1 were used.

**Table 1**

| | Manufacturer | Article No. |
|---|---|---|
| Glasgow minimal essential medium (G-MEM) | Invitrogen | 11710-035 |
| Gelatin | SIGMA | G2500 |
| 2-Mercaptoethanol (2-ME) | SIGMA | M7522 |
| Dulbecco's phosphate buffered saline, MgCl²⁽⁻⁾, CaCl²⁽⁻⁾ | Invitrogen | 14190-250 |
| L-glutamine, 200 mM | Invitrogen | 25030-081 |
| Non-essential amino acids (NEAA) | Invitrogen | 11140-050 |
| Fetal bovine serum (FBS) | EQUITECH | SFB30-960 |
| LIF | Chemicon | ESG1107 |
| 0.25% (w/v) trypsin-EDTA | Invitrogen | 25200-072 |
| KNOCKOUT serum replacement (KSR) | Invitrogen | 10828-028 |
| 6-cm dish | FALCON | 35 3802 |

The composition of the medium for maintaining embryonic stem cells was: 89% G-MEM, 1% FBS, 10% KSR, 10⁻⁴ M 2-ME, 2 mM L-glutamine, 0.1 mM NEAA, and 1000 U/ml LIF.

EB5 cells derived from Mouse 129 strain (Niwa H. et al., Genes and Development, 1998, 12:2048-2060) were used as embryonic stem cell.

### b. Procedures

Six-cm dishes were coated with gelatin. Coated dishes were seeded with 2 x 10⁵ EB5 embryonic stem cells. Next day, the medium was exchanged once. When the cells became confluent at day 2, the cells were detached from the dish using trypsin, and seeded again onto gelatin-coated dishes at a concentration of 2x10⁵ cells. Culture was carried out on a 5% CO₂ incubator at 37°C.

### Reference Example 2: Induction of the Differentiation of Embryonic Stem Cells in Serum-Free Medium

### a. Materials

For inducing the differentiation of embryonic stem cells, materials indicated in Table 2 were used.

**Table 2**

| | Manufacturer | Article No. |
|---|---|---|
| S-Clone (SF-03) | Sanko Junyaku Co., Ltd. | SS1303 |
| Bovine serum albumin (BSA) | SIGMA | A-2153 |
| 2-Mercaptoethanol (2-ME) | SIGMA | M7522 |
| Dulbecco's phosphate buffered saline, MgCl²⁽⁻⁾, CaCl²⁽⁻⁾ | Invitrogen | 14190-250 |
| BIOCOAT collagen IV-coated 10-cm dish | Becton-Dickinson | 35 4453 |
| Cell separation buffer | Invitrogen | 13150-016 |
| Activin A | Genzyme | 23389 |
| Anti-cadherin antibody (ECCD2) | Takara Shuzo Co., Ltd. | M108 |

### b. Medium for Differentiating Embryonic Stem Cells

To SF-03 powder was added one liter of distilled water and the accompanying NaHCO₃ (2.2 g) was added, and the mixture was stirred. Then, carbon dioxide gas was added into the medium until the color of the medium was changed to orange. BSA was added to a concentration of 0.1%, 2-ME was added to 10⁻⁴ M, and the mixture was stirred for more than 30 minutes. Finally, the solution was filter sterilized.

### c. Procedures for Differentiation Induction

BIOCOAT collagen IV-coated 10-cm dishes were seeded with 1x10⁵ EB5 embryonic stem cells. At day 3 to day 6, the cells were separated using a cell separation buffer (Invitrogen) and used for subsequent experiments.

### Reference Example 3: Selection of GFP-Positive Cells or GFP-Positive, E-Cadherin-Positive Cells by FacsVantage

### a. Preparation of Reagents

The regents indicated in Table 3 were used.

**Table 3**

| | Manufacturer | Article No. |
|---|---|---|
| 10x Hank's equilibrated salt solution (10x Hank's buffer) | Invitrogen | 14185-052 |
| Bovine serum albumin (BSA) | SIGMA | A-2153 |

To 900 ml of deionized water were added 100 ml of 10x Hank's buffer and 10g of BSA (final concentration of 1%), and the mixture was well stirred. After the BSA was dissolved, the solution was sterilized with a 0.2 µm filter. Hereinafter, the resultant solution is referred to as BSA-Hank's buffer.

### b. Procedures

Differentiation-induced embryonic stem cells at day 3 to day 6 of the differentiation were disaggregated with the cell separation buffer and then washed once with 1% BSA-Hank's buffer. Then, the cells were dissolved in 1 ml of 1% BSA-Hank's buffer per 10⁶ cells and GFP-positive cells were collected by cell selection. When antibody staining for E-cadherin was performed, 10 µl of mouse serum per 10⁶ cells was added and incubated for 20 minutes on ice to carry out blocking. A biotin-labeled antibody against E-cadherin was added and incubated on ice for 20 minutes. After 20 minutes, the cells were washed once with 1% BSA-Hank's buffer. The cells were re-dissolved in 500 µl of 1% BSA-Hank's buffer containing streptavidin-allopycocyanine (APC) and incubated on ice for 20 minutes. Finally, the cells were washed twice with 1% BSA-Hank's buffer, dissolved in 1 ml of 1% BSA-Hank's buffer per 10⁶ cells, and used for cell separation. In the case of the label dye being PE (phycoerythrin), similar results were obtained.

The usage of FacsVantage (Becton-Dickinson) was in accordance to the accompanying guidebook. The frequency of vibration of the nozzle was on the order of 26000, the level was at 3 V, and the drop delay was at about 12 to 14. Reference Example 4: Maintaining and Differentiating of Goosecoid-Positive Cells

For maintaining and differentiating goosecoid (Gsc)-positive cells, reagents and equipment indicated in Table 4 were used.

**Table 4**

| | Manufacturer | Article No. |
|---|---|---|
| Minimal essential medium α-medium | Invitrogen | 12571-063 |
| 2-Mercaptoethanol (2-ME) | SIGMA | M7522 |
| L-glutamine, 200 mM | Invitrogen | 25030-081 |
| Fetal bovine serum (FBS) | EQUITECH | US128311 |
| BIOCOAT collagen IV-coated 10cm dish | Becton-Dickinson | 35 4453 |

### b. Procedures

Cell-separated GFP-positive cells were seeded on BIOCOAT collagen IV-coated 10cm dishes.

### c. Medium for maintaining and differentiating Gsc-positive cells

The composition of the maintaining and differentiating medium was 90% α-MEM, 10% FBS, 5x10⁻⁵ M 2-ME, and 2 mM L-glutamine.

### Example 1

### Establishing of Goosecoid-GFP Knocked-in Embryonic Stem Cells

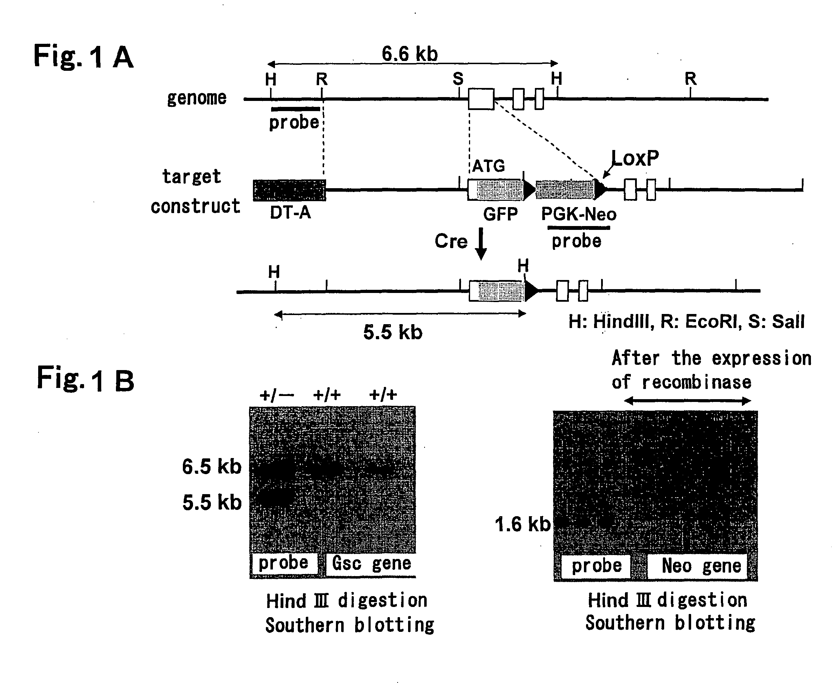

An construct into which a gene of green fluorescence protein (GFP) was introduced in frame to the start codon ATG of the mouse goosecoid gene, in accordance to routine procedures (for example, Gu H, Zou YR, Rajewsky K., Cell, 1993, 73, 1155-1164) was constructed and introduced into embryonic stem cells to obtain homologous recombinant clones (Fig. 1A).

Clones of interest were selected by southern blotting analysis using the GSC (goosecoid) gene as a probe. Normal cells in which homologous recombination has not occurred give a 6.5-kb-length fragment by HindIII digestion, whereas homologous recombination clones provide two fragments having lengths of 6.5 and 5.5 kb (Fig. 1B). Furthermore, southern blotting was carried out with an NEO probe, in order to identify whether the NEO gene was completely removed, and the disappearance of the signal was observed (Fig. 1B, right).

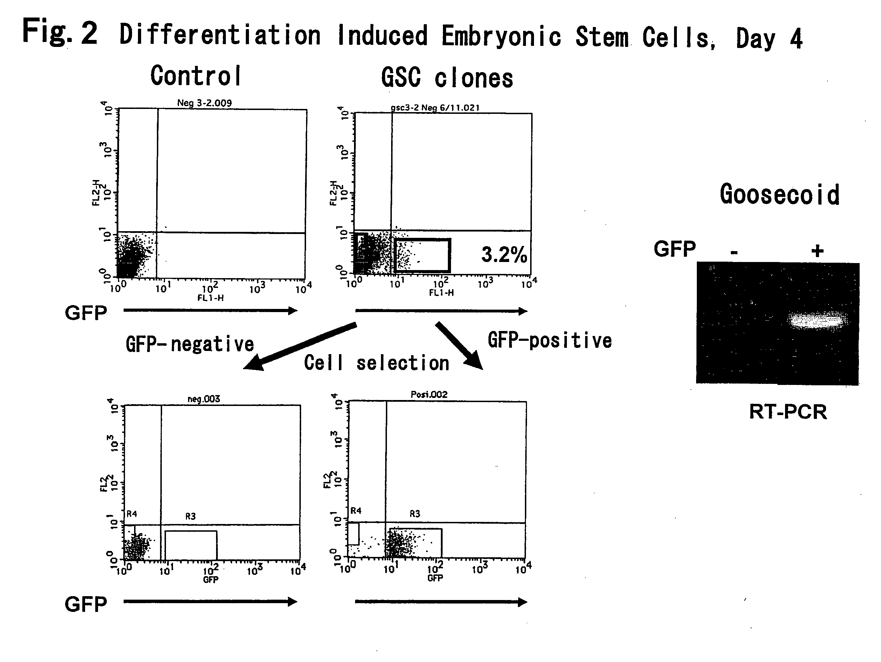

Embryonic stem cells treated as described above were added onto collagen IV-coated culture plates with the differentiating medium without LIF and supplemented with serum, and cultured at 37°C on a 5% CO₂ incubator. At day 4, about 3% GFP-positive cells were observed. These cells were subjected to cell separation employing FacsVantage and testing for the expression of the goosecoid gene by RT-PCR method. From the results, the expression of goosecoid was shown only in the GFP-positive cells (Fig. 2). This confirmed that the expression of GFP corresponded with the expression of goosecoid.

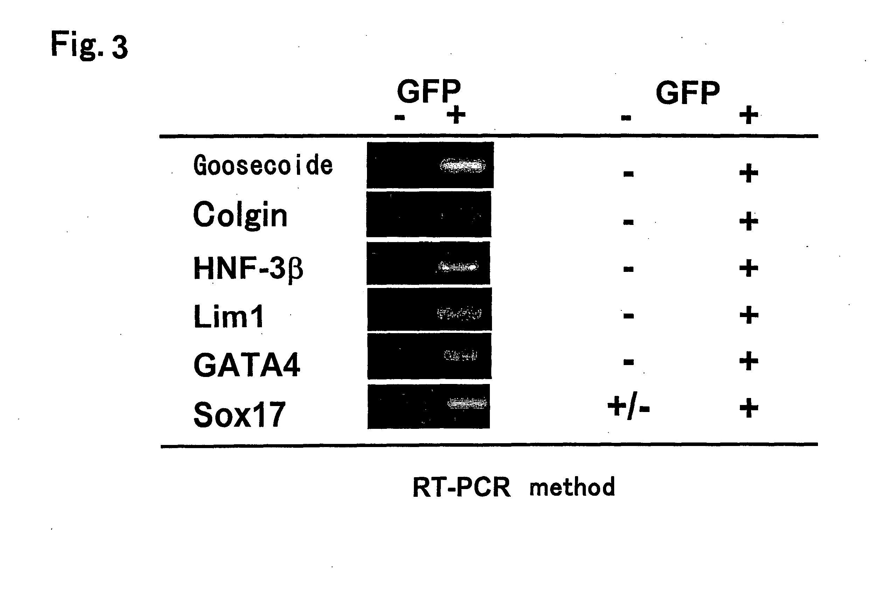

Next, whether these GFP-positive cells express other genes specific for node cells was examined using RT-PCR in similar ways (Fig. 3). Interestingly, these cells specifically express not only node cell-specific genes, but also endoderm-specific marker genes (GATA4, Soc17). The pattern of gene expression is similar to that of node cells and of their offspring cells. It turned out form these findings that the group of these cells contains so-called endomesodermal cells.

### Example 2

### Establishing of Conditions for Purifying GSC-Positive Cells

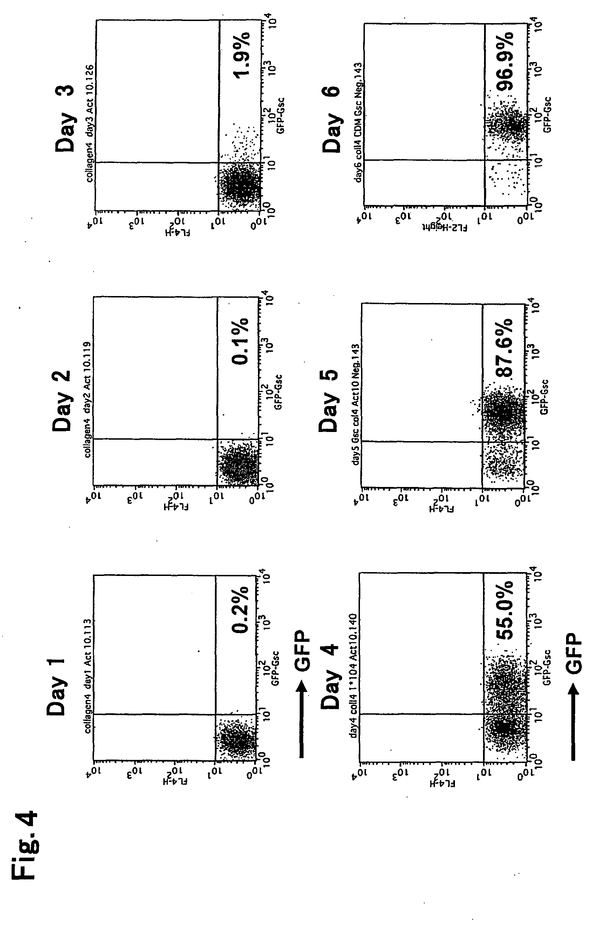

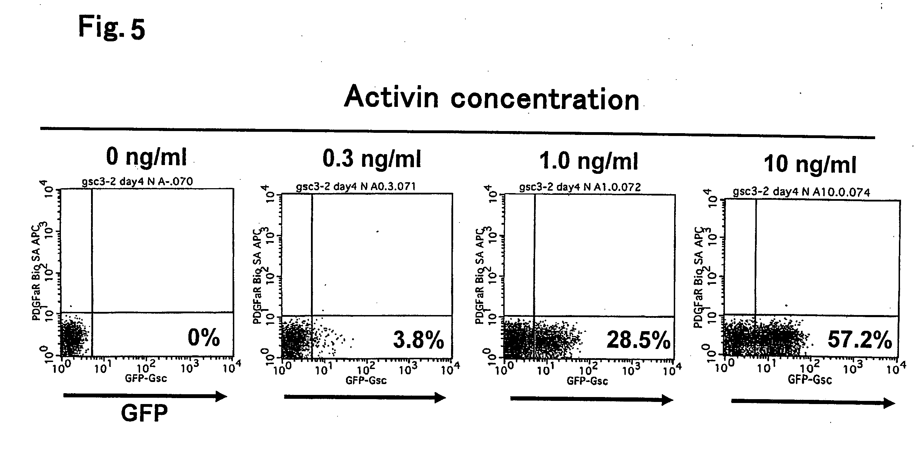

As shown in Example 1, GFP-positive cells appear at levels of 2% to 3% in the serum-supplemented medium, and their percentages are small. Examinations were firstly made by comparison as to whether any changes could result from the addition of activin A, BMP-4 (bone morphogenetic protein-4), BMP-2, LiCl, and others under conditions in the presence of serum. Only a small effect was seen with activin A, and no significant effect was detected. Investigation of culture conditions bringing about the appearance of GSC-GFP-positive cells was then carried out using serum-free medium, and comparison was made in a similar way between activin A, BMP-4, BMP-2, LiCl, and others. Specifically, on BIOCOAT collagen IV-coated dishes, goosecoid knocked-in embryonic stem cells were subjected to the induction of their differentiation using the differentiation inducing medium without LIF in the absence of serum. In this case, activin A was added at a concentration of 10 ng/ml from day 0. The expression of GFP from day 2 up to day 6 was analyzed using Facs. The results show that when activin A was added, the expression of GFP was induced in 97% of the cells at day 6 (Fig. 4). On the other hand, the expression of GFP was not induced for BMP-4, BMP-2, and LiCl. Moreover, experiments in which activin was added from day 0 at concentrations of 0, 0.3, 1, and 10 ng/ml were carried out in a similar way and the expression of GFP at day 4 was analyzed using Facs. It turned out from the results that the percentage of GFP-positive cells was increased in a manner dependent on the concentration of activin A (Fig. 5). The gene expression in the cells thus obtained was the same as that in GFP-positive cells obtained when serum was added: HNF-3β, which is a gene specific for node cells, Lim1, and GATA-4 and Sox17, which are endoderm-specific genes, were expressed.

Next, effects of BMP-4 and bFGF (basic fibroblast growth factor) on inducing the differentiation of GFP-positive cells were studied.

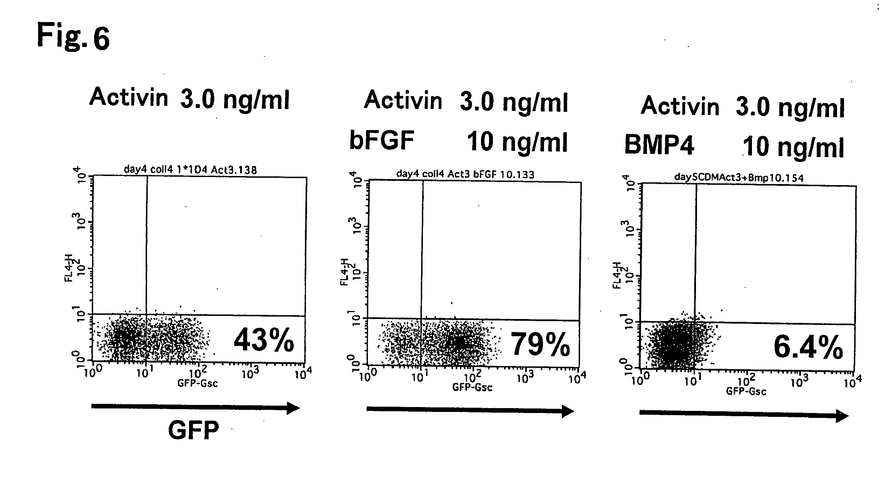

On BIOCOAT collagen IV-coated dishes, goosecoid knocked-in embryonic stem cells were subjected to the induction of their differentiation using the differentiation inducing medium without LIF in the absence of serum. In this case, activin A was added at a constant concentration of 3 ng/ml, and BMP-4 and bFGF at a concentration of 10 ng/ml. The addition of BMP-4 resulted in a reduced percentage of Gsc-GFP-positive cells and gave suppressive effects, whereas the addition of bFGF contrarily lead to an increased percentage, relative to the addition of activin A alone (Fig. 6). It is understood from these findings that the differentiation of GFP-positive cells are not induced by all growth factors and activin A and bFGF have said capability (although bFGF is auxially).

### Example 3

### Separation of Endodermal Cells from GFP-Positive Cells and

### Their Proliferation

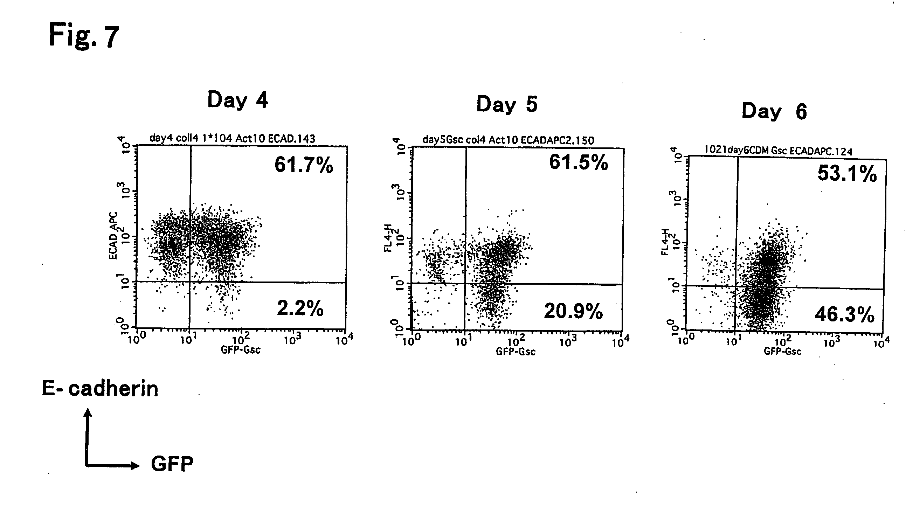

In order to analyze the differentiation capability of GFP-positive cells, analysis was made as to what changes in the pattern of expression of E-cadherin which is expressed in the endoderm and ectoderm were caused with the differentiation of embryonic stem cells. On BIOCOAT collagen IV-coated dishes, goosecoid knocked-in embryonic stem cells were subjected to the induction of their differentiation using the differentiation inducing medium without LIF in the absence of serum. In this case, activin A was added from day 0 at a concentration of 10 ng/ml. The expression of GFP and E-cadherin at days 4, 5, and 6, was analyzed using Facs. At day 4, most of the GFP-positive cells were E-cadherin positive. As the differentiation advances, GFP-positive, E-cadherin-negative cells appeared, and their percentages were increased (Fig. 7). These results, in the light of the expression of goosecoid in the endomesoderm, would indicate that GFP-positive, E-cadherin-positive cells are endodermal cells.

Next, attempts were made to differentiate into epithelioid cells from E-cadherin-positive and E-cadherin-negative cells among the GFP-positive cells.

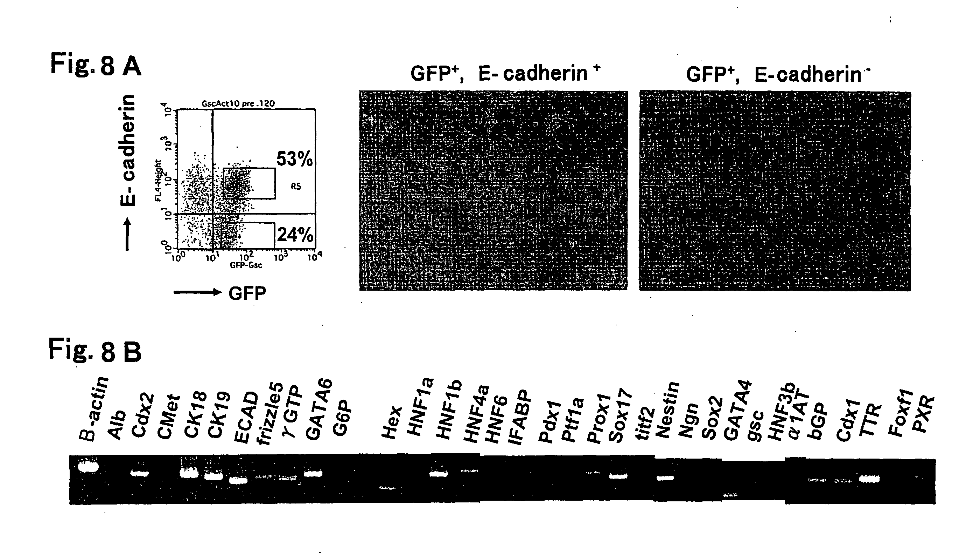

On BIOCOAT collagen IV-coated dishes, goosecoid knocked-in embryonic stem cells were subjected to the induction of their differentiation using the differentiation inducing medium without LIF in the absence of serum. In this case, activin A was added from day 0 at a concentration of 10 ng/ml. At day 6, respective cells which were GFP-positive/E-cadherin-positive and GFP-positive/E-cadherin-negative were stained with an antibody against E-cadherin and purified by cell selection employing FacsVantage. When these cells were cultured in the differentiating medium containing serum, the E-cadherin-positive fraction efficiently resulted in the appearance of only epithelioid cells, whereas the E-cadherin-negative fraction resulted in no observable appearance of epithelioid cells like these cells (Fig. 8A). This means that epithelioid cells appear preferentially from GFP-positive cell E-cadherin-positive cells.

Next, in order to investigate the cell linage of these epithelioid cells, the expression of genes specific for the cell linage was analyzed by RT-PCR method. The results showed that although HNF3b, which is an endoderm-specific marker, Sox17, and GATA4 were expressed, the expression of molecules expressed in hepatocytes and spleen cells was not detected (Fig. 8B). Cell morphology (Fig. 8A) and this gene expression pattern have shown that the epithelioid cells differentiated from GFP-positive, E-cadherin-positive cells at day 6 are endodermal cells.

### INDUSTRIAL APPLICABILITY

Endodermal stem cells according to the present invention differentiate into endodermal cells, the resulting endodermal cells are cells that differentiate into stomach, duodenum, intestinal tract, liver, spleen, and large bowel in the future. These endodermal cells can be utilized for development of drugs, or alternatively, as cells producing hormones, bioactive substances, and others, such as insulin producing cells, or for clinical applications.

## Claims

1. An endodermal stem cell which has been differentiated in vitro from a multipotential stem cell, wherein the cell is organizer-specific-marker positive and E-cadherin positive.

2. The cell according to claim 1, wherein the multipotential stem cell is an embryonic stem cell.

3. The cell according to claim 1 or 2, wherein the organizer-specific marker is goosecoid.

4. The cell according to claim 3, wherein a label protein is fused to the organizer-specific marker.

5. The cell according to claim 4, wherein the label protein is green fluorescence protein (GFP).

6. A method for preparing an endodermal stem cell, which comprises the steps of:
a) culturing multipotential stem cells, and
b) selecting and separating cells expressing an organizer-specific marker and E-cadherin.

7. The method according to claim 6, wherein a cell sorter is employed at the selection step.

8. The method according to claim 6 or 7, wherein the multipotential stem cells are cultured in the presence of activin in a serum-free medium on a culture plate coated with collagen IV, thereby differentiating the multipotential stem cells into endodermal stem cells.

9. A method for differentiating an endodermal stem cell according to any of claims 1 to 5 to prepare an endodermal cell of interest.

10. The method according to claim 9, wherein the endodermal stem cells are cultured in the presence of activin in a serum-free medium on a culture plate coated with collagen IV, thereby differentiating the endodermal stem cells into endodermal cells.

11. An endodermal cells obtained by the preparation method according to claim 9 or 10.

12. A method for preparing an intermediate stem cell of interest from a multipotential stem cell, which comprises the steps of:
a) introducing a gene of a label protein in the genome of a multipotential stem cell so as to be adapted to the expression system of a predetermined marker gene, such that said label protein is expressed instead of or together with the predetermined marker gene, and
b) selecting and separating intermediate stem cells of interest using the label of said label protein as an indicator.

13. The method according to claim 11, wherein the multipotential stem cell is an embryonic stem cell and the label protein is green fluorescence protein.

14. The method according to claim 12 or 13, wherein the intermediate stem cells are endodermal stem cells.
